# EUROPEAN PATENT SPECIFICATION

(11) **EP 0 886 729 B1**
(45) Date of publication and mention of the grant of the patent: **17.09.2003**
(21) Application number: 97914935.8
(22) Date of filing: 10.03.1997
(51) Int. Cl.: F04B 43/08

(54) **PERISTALTIC PUMP WITH PINCH FINGERS FOR PROVIDING COMPLETE OCCLUSION**
PERISTALTISCHE PUMPE MIT QUETSCHFINGERN ZUM SICHERSTELLEN DES VÖLLIGEN ABSCHLUSSES
POMPE PERISTALTIQUE A DOIGTS DE CONTACT ASSURANT L'OCCLUSION COMPLETE

(30) Priority: 12.03.1996 US 615704; 18.10.1996 US 731777; 18.11.1996 US 751548; 16.01.1997 US 784759
(43) Date of publication of application: 30.12.1998
(73) Proprietor: Moubayed, Ahmad-Maher, Mission Viejo, CA 92691 (US)
(72) Inventor: MOUBAYED, Ahmad-Maher, Mission Viejo, CA 92691 (US); JESTER, Robelio, B., Mexcali, Baja California (MX)
(74) Representative: MacGregor, Gordon
(86) International application number: US9703676
(87) International publication number: WO97034084

(56) References cited:
- DE-A- 2 152 352
- FR-A- 2 492 902
- SU-A- 853 157
- US-A- 2 393 838
- US-A- 3 518 033
- US-A- 3 778 195
- US-A- 5 092 749
- US-A- 5 217 355
- US-A- 5 575 631

## Description

### BACKGROUND OF THE INVENTION

This invention relates in general to fluid pumps and more specifically to a peristaltic pump with a cam driven plurality of fingers for sequentially engaging a resilient tube to create liquid flow through the tube and detectors for detecting occlusion of the tube at the input and output of the pump.

Conventional linear and rotary peristaltic pumps typically have a section of resilient tubing positioned between a wall and a set of rollers or reciprocating pushers that progressively compress sections of the tubing to pump liquids. Such pumps are often used in medical applications, such as intravenous infusion or withdrawing fluids such as in a wound drainage system. These pumps operate in a positive manner and are capable of generating substantial outlet pressures.

Typical linear peristaltic pumps include those described by Sorg et al. in U.S. Patent 2,877,714, Borsannyi in U.S. Patent No. 4,671,792 Heminway et al. in U.S. Patent No. 4,893,991 and Canon in U.S. Patent No. 4.728,265. While generally effective, these pumps are large, complex and cumbersome, requiring a drive shaft parallel to a resilient tube and a plurality of cams along the drive shaft to move pushers toward and away from the tube.

Rotary peristaltic pumps generally dispose a resilient tube along a circular path, with a number of rollers mounted around the circumference of a circular rotor sequentially rolling along the tube to occlude the tube and force liquid through the tube. Typical of such pumps are those disclosed by soderquist et al in US Patent No. 4,886,431 and Kling in US Patent No. 3,172,367. These pumps often have relatively low efficiency and impose high shear and tension stresses on the tube causing internal tube wall erosion or spallation. The tube may eventually be permanently deformed so that the tube becomes flattened into a more oval shape and carries less liquid.

SU-A-853157 discloses a peristaltic pump with rotated plungers made with ribs to increase its working life. It includes an elastic tube and plungers, each with a rib which compresses the tube. The plungers are reciprocated by the rotor's rollers. The plunger's body can be rotated around the rotor and fixed in position by a set screw. When the rotor is operated, its rollers move the plungers whose ribs compress the tube and create a wave which transfers the liquid to the receiver.

Another type of peristaltic pump has a tube arranged along a circular path with a cam member within the circle sequentially moving a plurality of blunt pushers or fingers outwardly to sequentially compress the tube from one end of the path to the other. Typical of these pumps are those shown by Gonner in German Patent No. 2,152,352 and Tubospir in Italian Patent No. 582,797.

These pumps tend to be less complex than linear peristaltic pumps. However, the pressure imposed by the blunt fingers reduces tube life, sometimes causing internal tube wall erosion or spallation, which results in particulate matter getting into the fluid stream. Tube with different wall thicknesses cannot be accommodated by these pumps, since with thinner than standard tubes the fingers will not properly occlude the tube and with thicker than standard tubes the tube will close prematurely and be subject to excessive compression, requiring higher cam drive power and causing excessive wear on the cam and tube.

In many applications of peristaltic pumps, in particular medical applications, it is important to promptly detect when the pump ceases to operate due to an occlusion in the pump tube either before or after the pump. An input occlusion occurring in the tube leading to the pump will cause the tube to collapse due to the fluid being sucked from the input side and pushed out the output side. An output occlusion occurring in the tube leading away from the pump will continue to push liquid into the output tube, inflating the tube and possibly causing it to burst. In either case, fluid flow to the end use is stopped.

These pumps tend to be less complex than linear peristaltic pumps. However, the pressure imposed by the blunt fingers reduces tube life, sometimes causing internal tube wall erosion or spallation, which results in particulate matter getting into the fluid stream. Tube with different wall thicknesses cannot be accommodated by these pumps, since with thinner than standard tubes the fingers will not properly occlude the tube and with thicker than standard tubes the tube will close prematurely and be subject to excessive compression, requiring higher cam drive power and causing excessive wear on the cam and tube.

Thus, there is a continuing need for both curvilinear and linear peristaltic pumps of greater simplicity, small size, low drive power requirements, which can accommodate resilient tubes of varying wall thickness while reducing wear and internal erosion of the resilient tube and which can automatically generate an emergency signal when either the input or output becomes occluded.

### SUMMARY OF THE INVENTION

The above-noted problems, and others, are overcome in accordance with this invention by a peristaltic pump having, in one embodiment, a concave curved, generally circular, platen for supporting a resilient tube, a multi-lobe cam rotatable about the center of the platen concavity, and a plurality of pump fingers riding on the cam as cam followers and guided to move in a radial direction toward and away from said platen.

In another embodiment, the pump has a generally planar platen for supporting a resilient tube, a plurality of parallel cams mounted on a rotatable shaft, a plurality of pump fingers, each riding on one of said cams.

Each pump finger has a face for engaging a tube on said circular or planar platen. Each face includes a narrow pinch finger spring centered in the face and biased to extend beyond the face.

Upon rotation of the cam or cams, the pump finger closest to the highest area on the corresponding cam in the direction of rotation will be moved outwardly in a radial direction to squeeze the tube against the platen. As the cam or cam assembly continues to rotate, the second pump finger will squeeze the tube as the pinch finger on the first pump finger occludes the tube, to force liquid in the tube to flow in the desired direction as the cam or cam assembly rotates. As cam rotation continues, the subsequent fingers will sequentially squeeze the tube to push liquid and then occlude the tube. At the same time, a pump finger just behind a lobe will move away from the tube, allowing the tube to expand and fill with liquid. This sequence continues as cam rotation proceeds.

In a second embodiment a pump having a rotary platen having pump fingers and pinch fingers, each pump finger has a face for engaging a tube on said circular platen. Each face includes a narrow pinch finger spring centered in the face and biased to extend beyond the face. Further, each pump finger includes a roller between the body of the pump finger and the cam to ride on the cam in the manner of a roller bearing, reducing wear.

When the cam is rotated, the pump finger closest to the highest area on the cam (widest lobe) in the direction of rotation will be moved outwardly in a radial direction to squeeze the tube against the platen. As the cam continues to rotate, the second pump finger will squeeze the tube as the pinch finger on the first pump finger occludes the tube, to force liquid in the tube to flow in the same direction as the cam rotates. As cam rotation continues, the subsequent fingers will sequentially squeeze the tube to push liquid and then occlude the tube. At the same time, the pump finger just behind the lobe will move away from the tube, allowing the tube to expand and fill with liquid. This sequence continues as cam rotation proceeds.

One embodiment of the pump includes pressure sensing means at the input and output ends of the pump for detecting collapse of the resilient tube due to an input occlusion and for detecting inflation of the resilient tube due to an output occlusion.

### BRIEF DESCRIPTION OF THE DRAWING

Details of the invention, and of preferred embodiments thereof, will be further understood upon reference to the drawing, wherein:
Figure 1 is a perspective view of the pump with the casing open and partially cut-away and one pump finger and pump finger mounted pinch finger cut-away;
Figure 2 is a side elevation of the pump at the beginning of a pumping cycle with the casing closed and the near side casing removed to show the internal components;
Figure 3 is a detail side elevation view of the pump finger assembly having a spring biased pinch finger and with the pinch finger partially cut-away;
Figure 4 is a detail side elevation view of a pump finger with an alternate pinch finger embodiment;
Figure 5 is a schematic side elevation view of the pump with input and output occlusion detection means;
Figure 6 is a side elevation view of a curvilinear pump having platen mounted pinch fingers;
Figure 7 is a detail side elevation view, partially cut away, of the pinch finger assembly having a spring biased pinch finger;
Figure 8 is a section view, taken on line 8--8 in Figure 7;
Figure 9 is a detail side elevation view of a fixed pinch finger tip;
Figure 10 is a side elevation view of a linear peristaltic pump with platen mounted pinch fingers;
Figure 11 is an elevation view, partially cut away, of a second embodiment of the pump of Figure 1, having a planar platen;
Figure 12 is a detail view, partially cut away, of one embodiment of a pinch finger useful with the pump of Figure 11; and
Figure 13 is a detail view of a second embodiment of a pinch finger useful with the pump of Figure 11.

### DETAILED DESCRIPTION OF PREFERRED EMBODIMENTS.

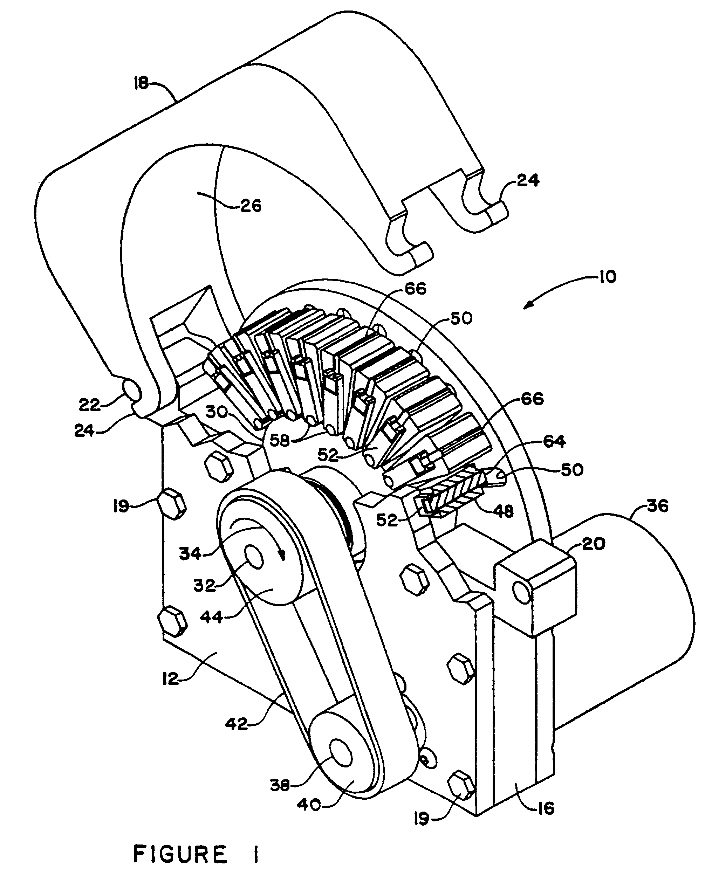

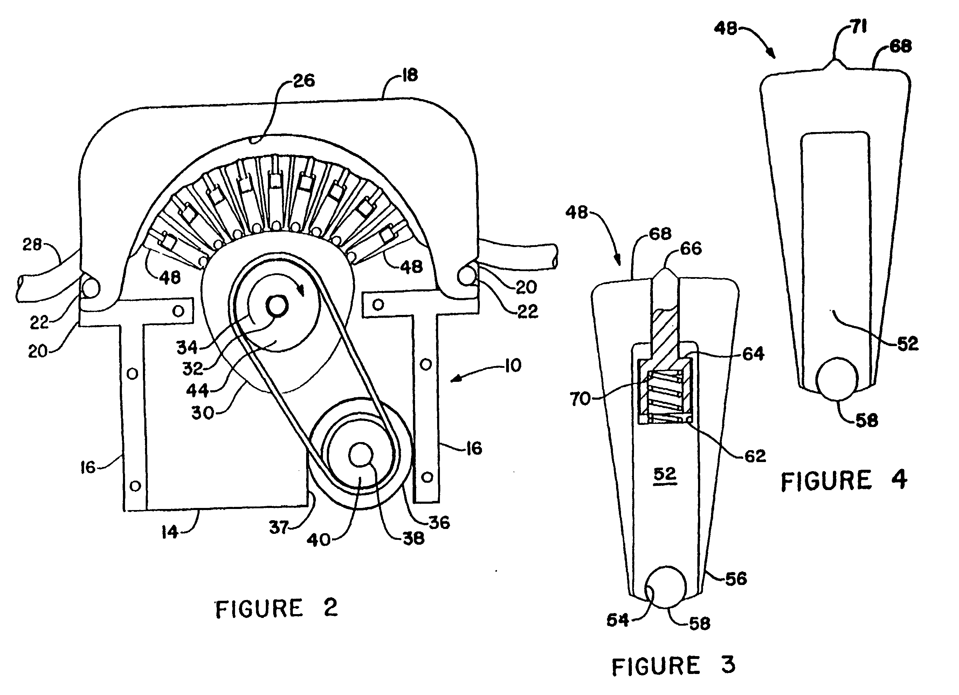

Referring to Figures 1 and 2, there is seen a curvilinear peristaltic pump 10 having a casing basically consisting of a front plate 12, a back plate 14 and spacers 16. The casing is held together by a plurality of bolts 19 for ease of assembly and disassembly as needed. A removable cover 18 is secured to casing 10. Each spacer 16 includes a block 20 having a hole therethrough cooperating with a pin or bolt 22 and hook-shaped cover extensions 24 to hold cover in place.

Cover 18 includes a concave curvilinear platen 26. While platen 26 may have any suitable surface, generally a cylindrical surface is preferred. As best seen in Figures 2 and 3, a resilient tube 28 may be laid along platen 26, exiting through the open space between each pair of extensions 24.

A multi-lobed cam 30 is mounted for rotation about an axle 32 that extends through suitable bearings in front and back plates 12 and 14. Cam 30 may have any suitable number of lobes, two or more. For optimum performance with smallest size, the three-lobe cam shown is preferred. Where platen 26 is cylindrical, axle 32 is preferably at the axis of the platen. Cam 30 can be rotated in either direction to pump liquid through tube 28 in either direction. For convenience of operation explanation, cam 30 will be considered to be rotating clockwise, as indicated by arrow 34. Any suitable drive means may be used to rotate cam 30. In the preferred embodiment shown, an electric drive motor 36 extends through opening 37 in back plate 14 and is mounted on the back surface of front plate 12. Motor 36 has a drive shaft 38 extending through front plate 12 to a pulley 40. A drive belt 42 extends from pulley 40 to pulley 44 mounted on cam axle 32. Pulleys 40 and 44 are sized to provide the desired cam rotation speed. A variable speed motor 36 may be used to allow cam rotation speed to be easily varied. If desired, a gear system could be used in place of belt 42, or a different drive system could be used, such as a conventional hydraulic drive, in place of the electric motor and belt drive system shown.

A plurality of pump fingers 48, as best seen in Figures 1 and 3, are mounted for radial movement on front plate 12 and back plate 14 between cam 30 and platen 26. Any suitable number of pump fingers 48 may be used. Where a greater number of cam lobes are used, fewer fingers will generally be used. On the other hand, if narrow fingers 48 are used, a larger number may be provided. A large scale pump will generally use a larger number of fingers. A preferred number of pump fingers 48 for a three-lobe cam 30 of maximum efficiency coupled with small size is from 7 to 11 pump fingers, with 9 generally being optimum. As seen in Figure 1, a plurality of opposed radial grooves 50 are provided in front plate 12 and back plate 14 to receive side extensions 52 that extend into grooves 50 and are freely movable therealong.

In the embodiment of Figures 1-4, a pinch finger assembly having a narrow pinch finger 66 is spring centered in the face and biased to extend beyond the face. Each pump finger 48, as best seen in Figure 3, includes a cylindrical recess 54 at a first end 56 for rotatably receiving a bearing roller 58. Rollers 58 freely roll on the surface of cam 30 in the manner of roller bearings, reducing wear on the cam surface. Side extensions 52 as seen in Figure 1 are formed on the sides of pumping finger 48. A transverse, inverted "T" slot 62 is formed across the top of pump finger 48. A base 64 mounting a transverse pinch finger 66 fits within slot 62, with pinch finger 66 extending through a transverse slot in the pumping surface along second end 68 of pump finger 48, as seen in Figure 1. A spring 70 biases base 64 and pinch finger 66 toward the extended position.

The pump operates in the following manner. As seen in Figure 2, two lobes of cam 30 are located at the beginning and end of the series of pump fingers 48. At this position, pump fingers 48 engaging the central portion of tube 28 along the middle of platen 26 are relatively withdrawn and those at the ends are relatively extended, thereby creating a zone of occlusion. Thus, the central portion of tube 28 is filled with liquid and the ends are substantially occluded. As cam 30 rotates in the direction of arrow 34, the second left pump finger 48 is pressed further against tube 28 while the rightmost pump finger begins to withdraw. Liquid is thus pushed in a zone of occlusion toward the right or outlet end of tube 28 and begins to exit. As cam rotation continues, pump fingers 48 are sequentially extended from the left and withdrawn at the right, forcing liquid in tube 28 toward the outlet end.

As seen in the central region of tube 28 in Figure 2, pinch fingers 66 under the forces of springs 70 are relatively extended. The leftmost pump finger 48 is slightly extended, but second end 68 of pump finger 48 has not entirely occluded tube 28. Pinch finger 48 is extended sufficiently under the force of spring 70 to occlude the tube. With a thin wall tube 28, pinch finger 48 will extend further to close the tube. With a thick walled tube, pinch finger will only extend a shorter distance until the tube is closed. Thus, only enough force is applied through the pinch finger to close the tube.

In prior art pumps, the pumping finger extended only a single preset distance under the strong mechanical force of a cam. With those arrangements, thin tubes are not entirely occluded and thick walled tubes are crushed beyond closure, often resulting in rapid wear, internal wall erosion and spallation with the resulting injection of particles of wall material into the liquid stream, of great concern in many infusion operations. Only a short degree of extension and retraction of pinch fingers 66 is required to produce this highly advantageous result, typically from about .2 to 1.0 mm.

Figure 4 shows a side elevation view of a second embodiment of pump fingers 48. Here the pump fingers 48 use a pinch finger in the form of a fixed transverse ridge 71 across the surface 68 of the pump fingers in place of the spring biased pinch fingers 66 of the embodiment of Figure 3. While the Figure 3 embodiment is generally preferred for lowest tube wear and the ability to work well tubes of slightly varying diameter and wall thickness, in other cases the lower cost version of Figure 4 may be preferred where the tube is more dimensionally uniform or the motor has sufficient power and the tube can take greater compression.

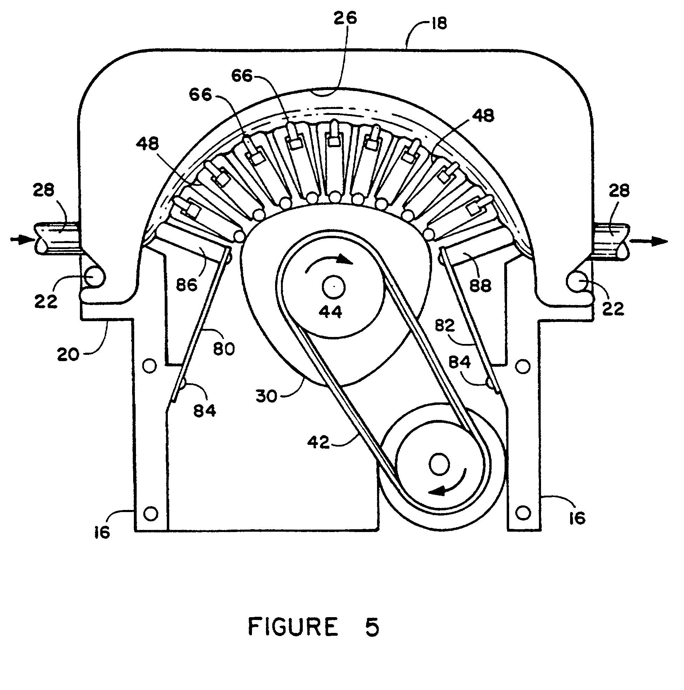

Figure 5 shows the pump of Figures 1-3 with a mechanism for detecting occlusion in either the input or output portions of tube 28. Strain gauge beams 80 and 82 are secured at their proximal ends to spacers 16 by bolts 84 or the like adjacent to the input and output ends, respectively, of tube 28. Any suitable strain gauge means, such as the strain gauge beams shown may be used, including any conventional strain gauge transducers may be used. Sensor members 86 and 88 on the distal end of beams 80 and 82 engage the input and output ends of tube 28, respectively. Stain gauges 80 and 82 operate in a conventional manner, generating an electrical signal proportional to the degree to which the beam is bent as tube 28 expands or contracts. The signal from strain gauge beams 80 and 82 can be directed to any suitable device for sounding an alarm, shutting down the pump, etc., as desired. Strain gauge beam 80 can thus generate a signal when the input portion of tube 28 collapses due to an upstream occlusion and strain gauge beam can generate a signal when the output portion of tube 28 expands due to a downstream occlusion.

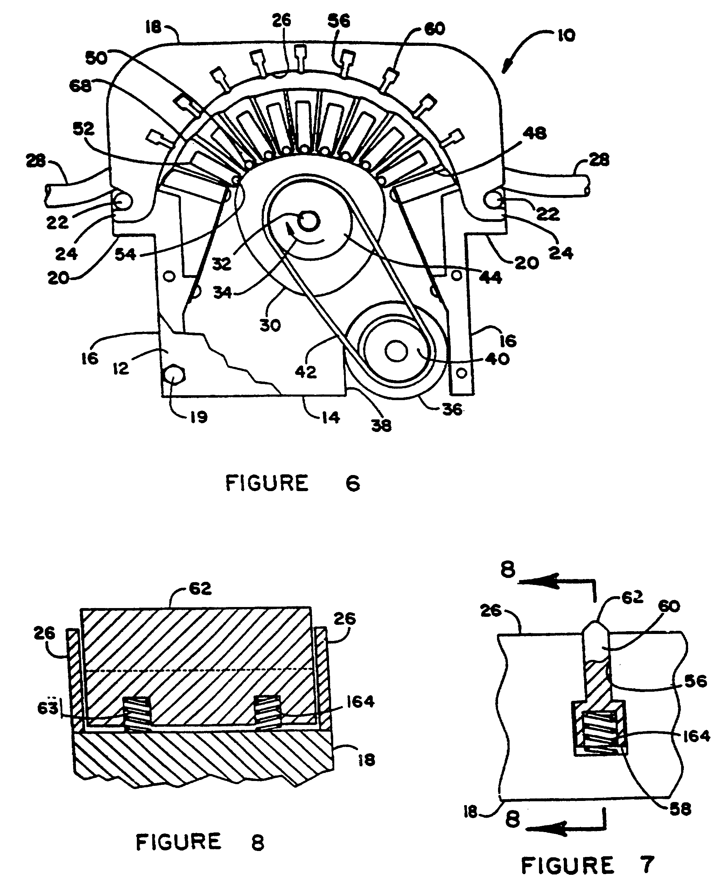

Referring to Figure 6, there is seen a curvilinear peristaltic pump 10 having a casing basically consisting of a back plate 14 (and corresponding front plate 12) and spacers 16. The casing is held together by a plurality of bolts 19 for ease of assembly and disassembly as needed. A removable cover 18 is secured to casing 10. Each spacer 16 includes a block 20 having a hole therethrough cooperating with a pin or bolt 22 and hook-shaped cover extensions 24 to hold cover 18 in place.

Cover 18 includes a concave curvilinear platen 26. While platen 26 may have any suitable surface, generally a cylindrical surface is preferred. A resilient tube 28 may be laid along platen 26, exiting through the open space between each pair of extensions 24.

A multi-lobed cam 30 is mounted for rotation about an axle 32 that extends through suitable bearings in front and back plates 12 and 14. Cam 30 may have any suitable number of lobes, two or more. For optimum performance with smallest size, the three-lobe cam shown is preferred. Where platen 26 is cylindrical, axle 32 is preferably at the axis of the platen. Cam 30 can be rotated in either direction to pump liquid through tube 28 in either direction. For convenience of operation explanation, cam 30 will be considered to be rotating clockwise, as indicated by arrow 34. Any suitable drive means may be used to rotate cam 30. In the preferred embodiment shown, an electric drive motor 36 extends through opening 38 in back plate 14 and is mounted on the back surface of front plate 12. Motor 36 has a drive shaft extending through front plate 12 to a pulley 40. A drive belt 42 extends from pulley 40 to pulley 44 mounted on cam axle 32. Pulleys 40 and 44 are sized to provide the desired cam rotation speed. A variable speed motor 36 may be used to allow cam rotation speed to be easily varied. If desired, a gear system could be used in place of belt 42, or a different drive system could be used, such as a conventional hydraulic drive, in place of the electric motor and belt drive system shown.

A plurality of pump fingers 48 are mounted for radial movement on front plate 12 and back plate 14 between cam 30 and platen 26. Any suitable number of pump fingers 48 may be used. Where a greater number of cam lobes are used, fewer fingers will generally be used. On the other hand, if narrow fingers 48 are used, a larger number may be provided. A large scale pump will generally use a larger number of fingers. A preferred number of pump fingers 48 for a three-lobe cam 30 of maximum efficiency coupled with small size is from 7 to 11 pump fingers, with 9 generally being optimum.

Preferably, a plurality of opposed radial grooves (not seen) are provided in back plate 14 and a corresponding front plate to receive side ridges 52 that extend into the corresponding grooves. The extension and ridge arrangement allows the pump fingers 48 to slide radially toward and away from axis 32 as the cam lobes gradually extend and retract against the pump fingers.

Each pump finger 48 includes a transverse cylindrical recess at a first end 50 for rotatably receiving a bearing roller 54. Rollers 54 freely roll on the surface of cam 30 in the manner of roller bearings, reducing wear on the cam surface.

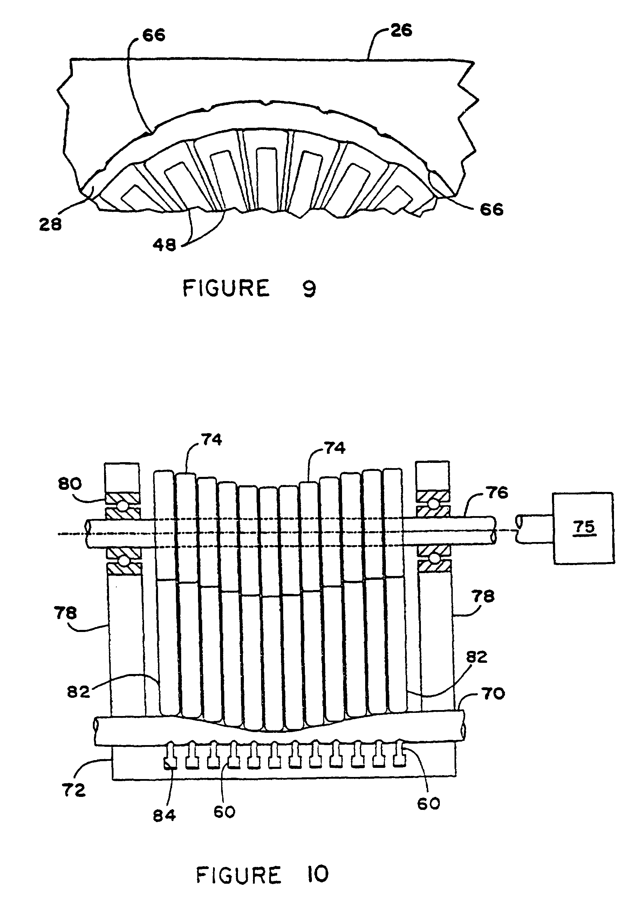

As detailed in Figures 6 and 7, a transverse, "T" shaped slot 56 is formed across cover 18 with the slot leg extending from platen 26. A base 58 fits within the crosspiece of "T" slot 56. A transverse pinch finger 60 extends from base 58 and fits with in the leg portion of "T" slot 56 and has a tip 62 extending out of the slot to extend above the platen surface. One or more springs 64 (preferably two, as seen in Figure 8) bias base 58 and tip 62 of pinch finger 60 toward the extended position.

The pump operates in the following manner. As seen in Figure 6, two lobes of cam 30 are located at the beginning and end of the series of pump fingers 48. At this position, pump fingers 48 engaging the central portion of tube 28 along the middle of platen 26 are relatively withdrawn and those at the ends are relatively extended, thereby creating a zone of occlusion. Thus, the central portion of tube 28 is filled with liquid and the ends are substantially occluded. As cam 30 rotates in the direction of arrow 34, the second left pump finger 48 is pressed further against tube 28 while the rightmost pump finger begins to withdraw. Liquid is thus pushed in a zone of occlusion toward the right or outlet end of tube 28 and begins to exit. As cam rotation continues, pump fingers 48 are sequentially extended from the left and withdrawn at the right, forcing liquid in tube 28 toward the outlet end.

As seen in the central region of tube 28 in Figure 6, pinch fingers 60 under the forces of springs 164 are relatively extended. The leftmost pinch finger 60 is slightly extended, but second end 68 of pump finger 48 has not entirely occluded tube 28. Pinch finger 60 is extended sufficiently, under the force of spring 164, opposite pump finger 48 to occlude the tube. With a thin wall tube 28, tip 62 of pinch finger 60 will extend further to occlude the tube. With a thick walled tube, tip 62 of pinch finger 60 will only extend a shorter distance until the tube is occluded. Thus, only enough force is applied through the pinch finger to occlude the tube.

In prior art pumps, the pumping finger extended only a single preset distance under the strong mechanical force of a cam. With those arrangements, thin tubes are not entirely occluded and thick walled tubes are crushed beyond closure, often resulting in rapid wear, internal wall erosion and spallation with the resulting injection of particles of wall material into the liquid stream, of great concern in many infusion operations. Only a short degree of extension and retraction of pinch fingers 60 is required to produce this highly advantageous result, typically from about .2 to 1.0 mm.

Figure 9 shows an alternative embodiment of the platen mounted pinch fingers that is useful with either the curvilinear platen of Figure 6 or the flat platen of Figure 10. Here, the pinch fingers are in the form of transverse tips or ridges 66 formed across the surface of platen 26 transverse to tube 28.

Pump fingers 48 are set such that when a pump finger is moved to the full distance toward tube 28, the pinch finger tips 66 will penetrate slightly more than would be required for full occlusion. Then, an undersized tube will still be fully occluded and an oversize tube will be penetrated by tips 66 to a slightly greater extent. Due to the small area and rounded ends on tips 66, significant damage to tube 28 is unlikely.

Tips 66 can be formed during manufacturing of platen 28 by conventional manufacturing methods, or may be secured as individual strips to the platen surface by welding, adhesive bonding, etc. While the embodiment of Figure 9 is simple and inexpensive to manufacture and generally very effective, for optimum tube life the embodiment of Figures 7 and 8 is preferred.

Figure 10 shows another pump embodiment in which the platen mounted pinch finger system of the invention can be used. Here,, a tube 70 rests on a flat platen 72. A set of cams 74 is mounted on an axle 76 arranged parallel to platen 72. Axle 76 is mounted on a uprights 78 through bearings 80. Axle 76 may be rotated by any suitable drive means, such as an electric motor (not shown). The cam assembly 74, drive, etc. are all conventional in the art. Typically each cam may be in the form of an off-center circle, ellipse, etc.

A pump finger 82 is mounted in a conventional support, allowing vertical movement relative to platen 72. As axle 76 is rotated, cams 74 progressively push pump fingers 82 down against tube 70 from one end toward the other, forcing liquid in tube 70 to move in that direction. As explained above, the occluding distance between each pump finger 82 and platen 72 is fixed so that a tube of precise predetermined wall thickness will be just barely fully occluded. If the walls of tube 70 are greater than normal, the tube will be crushed and damaged. If the tube walls are thinner than normal, full occlusion will not occur and back flow through the pump will occur, so that the expected pumping rate will not be achieved.

A plurality of pinch fingers 60 of the sort detailed in Figures 7 and 8 are arranged in slots 84 in platen 70, arranged transverse to tube 70. Of course, the pinch tip embodiment of Figure 4 may also be used. As described above, with a normal tube, the tips of pinch fingers 60 will be spring pressed toward tube 70 to extend slightly above the surface of platen 72 to fully occlude the tube. With a thinner walled tube, pinch fingers will be spring pressed to further extend above the platen surface, so that tube 70 is still occluded. Where the walls of tube 70 are oversize, the tips of pinch fingers 60 may only extend a shorter distance until the tube is occluded.

Thus, the platen mounted pinch fingers in both the embodiments of Figures 1 and 5 will accommodate a range of tube sizes without either damaging the tube or degrading pump performance.

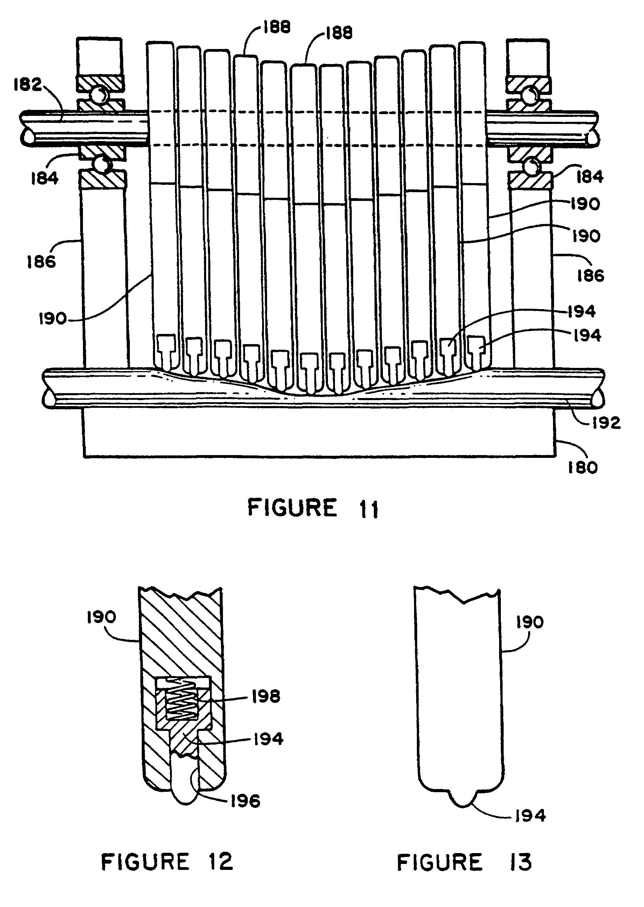

Figure 11 schematically illustrate a second embodiment of out peristaltic pump, having a linear platen 180. A shaft 182 is rotatably mounted through bearings 184 on posts 186 with the shaft generally parallel to platen 180.

A plurality of cams 188 are fixed to shaft 182 in a contiguous, parallel relationship. Cams 188 may have any suitable configuration. Typically, cams 188 could have a circular periphery with the shaft passing through each cam off center, could be an ellipse, could be multi-lobed as cam 30 in Figure 2, etc. The line passing through shaft 182 and the outermost portion of each cam is progressively radially offset through the set of cams.

Each cam 188 engages a pump finger 190 for longitudinal movement with the change in diameter of the corresponding cam. Each pump finger 190 may slide between front and back walls (not shown for clarity of illustration) of the sort shown in Figure 1 or any other suitable guide means. As each cam rotates, the corresponding pump finger 190 moves upwardly and downwardly in accordance with the cam surface. When a tube 192 is placed on platen 180, the pump fingers 190 will sequentially press against the tube to occlude the tube and progressively pump liquid through he tube in the manner described above.

As explained above, since tube wall thickness will vary, pressing a blunt pump finger 190 against a tube is likely to damage some tubes. To overcome this, a pinch finger 192 is used to fully occlude only a central area along each pump finger 190. Two embodiments of a suitable pinch finger are shown in detail view of Figures 12 and 13.

In the optimum embodiment of Figure 12, pinch finger 194 is slidably mounted in an "T" shaped slot 196 within an end of each pump finger 190. Pinch finger 194 has a corresponding shape and is movable a predetermined distance toward and away from the platen. A spring 198 presses pinch finger 194 toward platen 180. Thus, as a cam 188 moves the corresponding pump finger 190 toward tube 192, the tip of pinch finger 194 begins to occlude the tube. Once tube 192 is fully occluded, with the opposite tube walls in contact, pinch finger 194 is pushed back into pump finger 190 against spring 198 to prevent the tip from bing forced into the tube to the extent that the tube is damaged.

An alternate embodiment of pinch finger 194 is illustrated in the detail view of Figure 13. In this embodiment, pinch finger 194 is in the form of a ridge across the end of pump finger. As this pump finger is moved toward a tube 192 by the corresponding cam 188, pinch finger occludes the tube along a transverse line. While minor damage may occur if pinch finger 194 is pressed too far into tube 192, the damage will be less than if an entire blunt pump finger end was similarly overly pressed against the tube. Where tubes have consistent wall thicknesses, this embodiment provides excellent results. With tubes of varying wall thickness, the embodiment of Figure 12 is optimum.

While certain specific relationships, materials and other parameters have been detailed in the above description of preferred embodiments, those can be varied, where suitable, with similar results. Other applications, variations and ramifications of the present invention will occur to those skilled in the art upon reading the present disclosure. Those are intended to be included within the scope of this invention as defined in the appended claims.

## Claims

1. A peristaltic pump (10) which comprises:
a platen (26);
a rotatable cam means (30) spaced from said platen 26); means for rotating said cam means (30) in a first direction;
a plurality of pump fingers (48), each having a first end riding on said cam means (30) and a second end adjacent to said platen (26);
guide means for axially guiding said pump fingers (48);
said cam means (30) configured to first sequentially move said pump fingers (48) toward said platen (26) and second sequentially allow said pump fingers (48) to move away from said platen (26);
each of said pump fingers (48) having a pinch finger (66) extending beyond said second end, and extending transversely across said second end;
whereby a resilient tube (28) may be interposed between said platen (26) and said second pump finger ends (48) so that as said pump fingers (48) are sequentially moved toward said platen (26), liquid in said tube (28) will be pumped in said first cam rotation direction and each pinch finger (48) will occlude said tube (28) when each said pump finger (48) reaches a position closest to said platen (26), **characterised in that** each of said pump finger assemblies includes a pinch finger (66) extending beyond said second end, each said pinch finger (66) comprises a slidable member extending through a transverse slot (56) in each pump finger second end and further including means for biasing each said pinch finger in a direction extending outwardly of said transverse slot (56).

2. The peristaltic pump (10) of Claim 1 wherein said pump is a rotary pump having a curved, concave platen (26).

3. The peristaltic pump (10) of Claim 1 wherein said pump fingers (48) move in a radial direction.

4. The peristaltic pump (10) of Claim 1 wherein said pump is a linear pump having a generally planar platen (26).

5. The peristaltic pump (10) of Claim 1 further comprising a strain gauge (80) means engaging said tube (28) adjacent to each end of said set of pump finger assemblies to generate an electrical signal corresponding to the degree of inflation of said tube (28) at the strain gauge (80) locations.

6. The peristaltic pump (10) according to Claim 1 wherein each of said pump fingers (48) has a transverse cavity communicating with said transverse slot (56), a base member (58) within said cavity supporting a pinch finger (66) in said transverse slot (56), with a compression spring (64) between said base member (58) and a wall of. said cavity opposite said pinch finger (66).

7. The peristaltic pump (10) according to Claim 1 further including a pinch finger assembly mounted in a cavity in said platen (26) opposite each pump finger (48), each said pinch finger assembly comprises a slidable pinch finger extending beyond said platen (26) toward said opposite pump finger (48) and further including means for biasing each said pinch finger (66) toward said opposite pump finger (48).

8. The peristaltic pump (10) according to Claim 1 wherein each pump finger (48) carries a rotatable roller (54) at said first end to engage and roll along said cam means (30).

9. The peristaltic pump (10)according to Claim 1 further including releasable latch means for attaching said platen (26) to a cam means support casing.

10. The peristaltic pump (10) according to Claim 1 wherein said cam means (30) is supported for rotation between parallel front (12) and back plates (14) and further including guide means comprising co-operating pairs of radial grooves (50) in said front and back plates (12, 14) and further including side extensions (52) on each pump finger (48) for radial sliding in a pair of said grooves (50).

11. A peristaltic pump (10) according to Claim 2 wherein said cam means (30) is a single cam.

12. A peristaltic pump (10) according to Claim 3 wherein said cam means (30) is a rotatable cam assembly spaced from said platen (26).

13. A peristaltic pump (10) according to Claim 3 wherein said cam assembly comprising a plurality of contiguous cams mounted on a shaft (38) with lobes of said cams (30) sequentially radially offset along said shaft (38).

14. The peristaltic pump (10) according to Claim 4 wherein each said pinch finger (66) comprises a slidable member extending through a transverse slot (56) in each pump finger (48) second end and further including means for biasing each said pinch finger (66) in a direction extending outwardly of said transverse slot (56).

15. The linear peristaltic pump (10)according to Claim 4 wherein each said pinch finger (66) is a transverse ridge across each said pump finger (48) second end.

16. The linear peristaltic pump (10) according to Claim 4 wherein each of said pump fingers (48) has a transverse cavity communicating with said transverse slot (56), a base member (58) within said cavity supporting a pinch finger (66) in said transverse slot (56), with a compression spring (64) between said base member(58)and a wall of said cavity opposite said pinch finger (66).

17. The peristaltic pump (10) according to Claim 2 having a pinch finger tip (66) mounted on said platen (26) opposite each of said pump fingers (48), each tip extending above the surface of said platen (26) transverse to a tube (28) in place on said platen (26).

18. The peristaltic pump (10) according to Claim 17 wherein each of said tips is mounted on a pinch finger (66) slidably mounted in a slot in said platen (26) opposite each said pump finger (48);
each of said tips being extendable out of said slot a predetermined maximum distance toward said opposite pump finger (48); and
biasing means for urging each of said pinch fingers (66) to extend each said tip out of each said slot.

## Patentansprüche

1. Peristaltische Pumpe (10), die umfasst:
eine Platte (26),
ein drehbares Nocken- bzw. Kurvensteuermittel (30), das von der Platte (26) beabstandet ist,
Mittel zum Drehen des Nockenmittels (30) in einer ersten Richtung,
mehrere Pumpenfinger (48), von denen jeweils ein erstes Ende auf dem Nockenmittel (30) gleitet und ein zweites Ende an die Platte (26) angrenzt,
Führungsmittel zum axialen Führen der Pumpenfinger (48),
wobei das-Nockenmittel (30) so konfiguriert ist, dass es zunächst die Pumpenfinger (48) nacheinander zu der Platte (26) hin bewegt und anschließend die Pumpenfinger (48) sich nacheinander von der Platte (26) wegbewegen lässt,
wobei jeder der Pumpenfinger (48) einen Klemmfinger (66) aufweist, der sich über das zweite Ende hinaus erstreckt und sich quer über das zweite Ende erstreckt,
wodurch ein federndes oder elastisches Rohr (28) zwischen die Platte (26) und die zweiten Enden der Pumpenfinger (48) eingefügt werden kann, so dass, wenn die Pumpenfinger (48) nacheinander zu der Platte (26) hin bewegt werden, Flüssigkeit in dem Rohr (28) in der ersten Nockendrehrichtung gepumpt werden wird und jeder Klemmfinger (48) das Rohr (28) verschließen wird, wenn jeder Pumpenfinger (48) eine der Platte (26) nächstgelegene Position erreicht,
**dadurch gekennzeichnet, dass** jede der Pumpenfingeranordnungen einen Klemmfinger (66) aufweist, der sich über das zweite Ende hinaus erstreckt, wobei jeder Klemmfinger (66) ein verschiebbares Element umfasst, das sich durch einen Querschlitz (56) in jedem zweiten Pumpenfinger-Ende erstreckt und ferner Mittel zum Vorbelasten jedes Klemmfingers in einer sich vom Querschlitz (56) nach außen ertreckenden Richtung aufweist.

2. Peristaltische Pumpe (10) nach Anspruch 1, wobei die Pumpe eine Rotationspumpe mit einer gekrümmten, konkaven Platte (26) ist.

3. Peristaltische Pumpe (10) nach Anspruch 1, wobei sich die Pumpenfinger (48) in einer Radialrichtung bewegen.

4. Peristaltische Pumpe (10) nach Anspruch 1, wobei die Pumpe eine lineare Pumpe mit einer im allgemeinen planaren Platte (26) ist.

5. Peristaltische Pumpe (10) nach Anspruch 1, ferner mit einem Dehnungsmessmittel (80), das mit dem Rohr (28) angrenzend an jedes Ende des Satzes von Pumpenfingeranordnungen in Eingriff steht, um ein elektrisches Signal zu erzeugen, das dem Füllungsgrad des Rohrs (28) an den Dehnungsmessstellen (80) entspricht.

6. Peristaltische Pumpe (10) nach Anspruch 1, wobei jeder der Pumpenfinger (48) eine transversale Ausnehmung aufweist, die mit dem Querschlitz (56) in Verbindung steht, wobei ein Basiselement 58 in der Ausnehmung einen Klemmfinger (66) in dem Querschlitz (56) haltert, wobei sich eine Druckfeder (64) zwischen dem Basiselement (58) und einer Wand der Ausnehmung gegenüber dem Klemmfinger (66) befindet.

7. Peristaltische Pumpe (10) nach Anspruch 1, ferner mit einer Klemmfingeranordnung, die in einer Ausnehmung in der Platte (26) gegenüber jedem Pumpenfinger (48) angebracht ist, wobei jede Klemmfingeranordnung einen verschiebbaren Klemmfinger umfasst, der sich über die Platte (26) hinaus zu dem gegenüberliegenden Pumpenfinger (48) hin erstreckt, und ferner mit Mitteln zum Vorbelasten jedes Klemmfingers (66) zu dem gegenüberliegenden Pumpenfinger(48) hin.

8. Peristaltische Pumpe (10) nach Anspruch 1, wobei jeder Pumpenfinger (48) eine drehbare Rolle bzw. Walze (54) an dem ersten Ende trägt, um mit dem Nockenmittel (30) in Eingriff zu kommen und entlang diesem abzurollen.

9. Peristaltische Pumpe (10) nach Anspruch 1, ferner mit einem lösbaren Verriegelungsmittel zum Befestigen der Platte (26) an einem Nockenmittel-Lagergehäuse.

10. Peristaltische Pumpe (10) nach Anspruch 1, wobei das Nockenmittel (30) für eine Drehung zwischen parallelen vorderen (12) und hinteren Platten (14) gehaltert bzw. gelagert ist und ferner Führungsmittel aufweist, welche zusammenwirkende Paare von Radialnuten bzw. -rillen (50) in den vorderen und hinteren Platten (12,14) umfasst, und ferner seitliche Verlängerungen (52) an jedem Pumpenfinger (48) zum radialen Gleiten in einem Paar der Nuten bzw. Rillen (50) aufweist.

11. Peristaltische Pumpe (10) nach Anspruch 2, wobei das Nockenmittel (30) eine einzelne Nocke bzw. Steuerkurve ist.

12. Peristaltische Pumpe (10) nach Anspruch 3, wobei das Nockenmittel (30) eine drehbare Nocken- bzw. Kurvensteueranordnung ist, die von der Platte (26) beabstandet ist.

13. Peristaltische Pumpe (10) nach Anspruch 3, wobei die Nockenanordnung mehrere aneinandergrenzende Nocken umfasst, die an einer Welle (38) angebracht sind, wobei Erhebungen bzw. Höcker bzw. Steuerkurven der Nocken (30) nacheinander radial entlang der Welle (38) versetzt sind.

14. Peristaltische Pumpe (10) nach Anspruch 4, wobei jeder Klemmfinger (66) ein verschiebbares Element umfasst, das sich durch einen Querschlitz (56) in jedem zweiten Pumpenfinger-Ende (48) erstreckt und ferner Mittel zum Vorbelasten jedes Klemmfingers (66) in einer sich von dem Querschlitz (56) nach außen erstreckenden Richtung aufweist.

15. Lineare peristaltische Pumpe (10) nach Anspruch 4, wobei jeder Klemmfinger (66) ein Quersteg über jedem zweiten Pumpenfinger-Ende (48) ist.

16. Lineare peristaltische Pumpe (10) nach Anspruch 4, wobei jeder Pumpenfinger (48) eine transversale Ausnehmung aufweist, die mit dem Querschlitz (56) in Verbindung steht, ein Basiselement (58) in der Ausnehmung einen Klemmfinger (66) in dem Querschlitz (56) haltert, wobei sich eine Durckfeder (64) zwischen dem Basiselement (58) und einer Wand der Ausnehmung gegenüber dem Klemmfinger (66) befindet.

17. Peristaltische Pumpe (10) nach Anspruch 2, mit einer Klemmfingerspitze (66), die an der Platte (26) gegenüber jedem der Pumpenfinger (48) angebracht ist, wobei sich jede Spitze über die Oberfläche der Platte (26) quer zu einem auf der Platte (26) angeordneten Rohr (28) erstreckt.

18. Peristaltische Pumpe (10) nach Anspruch 17, wobei jede der Spitzen an einem Klemmfinger (66) angebracht ist, der verschiebbar in einem Schlitz in der Platte (26) gegenüber jedem Pumpenfinger (48) angebracht ist,
wobei jede der Spitzen aus dem Schlitz um eine vorbestimmte maximale Strecke zu dem gegenüberliegenden Pumpenfinger (48) hin ausstreckbar ist, und
Vorbelastungsmittel zum Vorbelasten jedes der Klemmfinger (66), um jede Spitze aus jedem Schlitz auszustrecken, vorgesehen sind.

## Revendications

1. Pompe péristaltique (10) qui comporte :
un plateau (26),
des moyens formant came rotative (30) espacés dudit plateau (26), des moyens pour faire tourner lesdits moyens formant came (30) dans une première direction,
une pluralité de doigts de pompe (48), ayant chacun une première extrémité montant sur lesdits moyens formant came (30) et une seconde extrémité adjacente audit plateau (26),
des moyens de guidage pour guider axialement lesdits doigts de pompe (48),
lesdits moyens formant came (30) étant configurés tout d'abord pour déplacer séquentiellement lesdits doigts de pompe (48) en direction dudit plateau (26), et deuxièmement pour permettre séquentiellement auxdits doigts de pompe de s'éloigner dudit plateau (26),
chacun desdits doigts de pompe (48) ayant un doigt de pincement (66) s'étendant au-delà de ladite seconde extrémité, et s'étendant transversalement à travers ladite seconde extrémité,
un tube souple (28) pouvant être interposé entre ledit plateau (26) et lesdites secondes extrémités de doigts de pompe (48), de sorte que lesdits doigts de pompe (48) sont déplacés séquentiellement en direction dudit plateau (26), un liquide situé dans ledit tube (28) va être pompé dans ladite première direction de rotation de came, et chaque doigt de pincement (48) va obturer ledit tube (28) lorsque chaque doigt de pompe (48) atteint une position la plus proche dudit plateau (26), **caractérisé en ce que** chacun desdits ensembles de doigts de pompe comporte un doigt de pincement (66) s'étendant au-delà de la dite seconde extrémité, chaque doigt de pincement (66) comporte un élément pouvant coulisser s'étendant à travers une fente transversale (56) située dans chaque seconde extrémité de doigt de pompe, et comportant de plus des moyens pour rappeler chaque doigt de pincement dans une direction s'étendant à l'extérieur de ladite fente transversale (56).

2. Pompe péristaltique (10) selon la revendication 1, dans laquelle chaque pompe est une pompe rotative ayant un plateau concave incurvé (26).

3. Pompe péristaltique (10) selon la revendication 1, dans laquelle lesdits doigts de pompe (48) se déplacent dans une direction radiale.

4. Pompe péristaltique (10) selon la revendication 1, dans laquelle ladite pompe est une pompe linéaire ayant un plateau globalement plan (26).

5. Pompe péristaltique (10) selon la revendication 1, comportant de plus des moyens formant jauge de contraintes (80) venant en prise avec ledit tube (28) de manière adjacente à chaque extrémité dudit ensemble d'ensembles de doigts de pompe pour générer un signal électrique correspondant au degré de gonflement dudit tube (28) au niveau des emplacements de la jauge de contraintes (80).

6. Pompe péristaltique (10) selon la revendication 1, dans laquelle chacun desdits doigts de pompe (48) a une cavité transversale communiquant avec ladite fente transversale (56), un élément de base (58) situé dans ladite cavité supportant un doigt de pincement (66) dans ladite fente transversale (56), un ressort de compression (64) étant situé entre ledit élément de base (58) et une paroi de ladite cavité opposée audit doigt de pincement (66).

7. Pompe péristaltique (10) selon la revendication 1, comportant de plus un ensemble de doigts de pincement monté dans une cavité située dans ledit plateau (26) opposé à chaque doigt de pompe (48), chaque ensemble de doigts de pompe comportant un doigt de pincement pouvant coulisser s'étendant au-delà dudit plateau (26) en direction dudit doigt de pompe opposé (48), et comportant de plus des moyens pour rappeler chaque doigt de pincement (66) en direction dudit doigt de pompe opposé (48).

8. Pompe péristaltique (10) selon la revendication 1, dans laquelle chaque doigt de pompe (48) supporte un rouleau rotatif (54) au niveau de ladite première extrémité, pour venir en contact avec lesdits moyens formant came (30), et rouler le long de ceux-ci.

9. Pompe péristaltique (10) selon la revendication 1, comportant de plus des moyens de verrouillage libérables pour fixer ledit plateau (26) sur un boîtier de support de moyens formant came.

10. Pompe péristaltique (10) selon la revendication 1, dans laquelle lesdits moyens formant came (30) sont supportés pour rotation entre des plaques avant (12) et arrière (14) parallèles, et comportent de plus des moyens de guidage comportant des paires coopérantes de gorges radiales (50) situées dans lesdites plaques avant et arrière (12, 14), et comportant de plus des prolongements latéraux (52) sur chaque doigt de pompe (48) pour coulisser radialement dans une paire desdites gorges (50).

11. Pompe péristaltique (10) selon la revendication 2, dans laquelle lesdits moyens formant came (30) sont constitués d'une came unique.

12. Pompe péristaltique (10) selon la revendication 3, dans laquelle lesdits moyens formant came (30) sont constitués d'un ensemble de came rotative espacé dudit plateau (26).

13. Pompe péristaltique (10) selon la revendication 3, dans laquelle ledit ensemble de came comportant une pluralité de cames contigües montées sur un arbre (38), des lobes desdites cames(30) étant décalés séquentiellement et radialement le long dudit arbre (38).

14. Pompe péristaltique (10) selon la revendication 4, dans laquelle chaque doigt de pincement (66) comporte un élément pouvant coulisser s'étendant à travers une fente transversale (56) située dans chaque seconde extrémité de doigt de pompe (48), et comportant de plus des moyens pour rappeler chaque dit doigt de pincement (66) dans une direction s'étendant à l'extérieur de ladite fente transversale (56).

15. Pompe péristaltique linéaire (10) selon la revendication 4, dans laquelle chaque doigt de pincement(66) est une crête transversale à travers chaque seconde extrémité de doigt de pompe (48).

16. Pompe péristaltique linéaire (10) selon la revendication 4, dans laquelle chacun desdits doigts de pompe (48) a une cavité transversale communiquant avec ladite fente transversale (56), un élément de base (58) situé dans ladite cavité supportant un doigt de pincement (66) dans ladite fente transversale (56), un ressort de compression (64) étant situé entre ledit élément de base (58) et une paroi de ladite cavité opposée audit doigt de pincement (66).

17. Pompe péristaltique (10) selon la revendication 2, ayant une pointe de doigt de pincement (66) montée sur ledit plateau (26) opposée à chacun desdits doigts de pompe (48), chaque pointe s'étendant au-dessus de la surface dudit plateau (26) transversalement par rapport à un tube (28) en place sur ledit plateau.

18. Pompe péristaltique (10) selon la revendication 17, dans laquelle chacune desdites pointes est montée sur un doigt de pincement (66) monté de manière coulissante dans une fente située dans ledit plateau (26) en étant opposé à chaque doigt de pompe (48),
chacune desdites pointes pouvant être étendue en dehors de ladite fente sur une distance maximale prédéterminée en direction dudit doigt de pompe opposé (48), et
des moyens de rappel pour pousser chacun desdits doigts de pincement (66) à étendre chaque dite pointe en dehors de chaque dite fente.
